# FASCICULE DE BREVET EUROPEEN

(11) **EP 2 699 678 B1**
(45) Date de publication et mention de la délivrance du brevet: **08.02.2017**
(21) Numéro de dépôt: 12717268.2
(22) Date de dépôt: 19.04.2012
(51) Int. Cl.: C12N 9/64, C07K 1/36, A61K 38/48, C07K 1/34

(54) **PROCEDE DE PREPARATION D'UN CONCENTRE DE FACTEUR XI**
VERFAHREN ZUR HERSTELLUNG EINES FAKTOR-XI KONZENTRATS
METHOD FOR PREPARING A CONCENTRATE OF FACTOR XI

(30) Priorité: 20.04.2011 FR 1153437
(43) Date de publication de la demande: 26.02.2014
(73) Titulaire: Laboratoire Français du Fractionnement et des Biotechnologies, 91940 Les Ulis (FR)
(72) Inventeur: MARTIN, Jean-François, F-59800 Lille (FR)
(74) Mandataire: Pontet Allano & Associes
(86) Numéro de dépôt international: PCT/EP2012/057227
(87) Numéro de publication internationale: WO 2012/143483

(56) Documents cités:
- EP-A1- 0 512 883
- BURNOUF-RADOSEVICH M ET AL: "A therapeutic, highly purified factor XI concentrate from human plasma", TRANSFUSION (BETHESDA), vol. 32, no. 9, 1992, pages 861-867, XP009084439, ISSN: 0041-1132

## Description

### DOMAINE DE L'INVENTION

La présente invention concerne un procédé de préparation du Facteur XI à partir de cryosurnageant de précipitation de plasma comprenant une étape d'adsorption-filtration et une étape de chromatographie sur résine échangeuse de cations.

### ETAT DE LA TECHNIQUE

Le Facteur XI ou précurseur de la thromboplastine plasmatique est une glycoprotéine qui fait partie de la phase de contact, dans le mécanisme de l'hémostase, par son action activatrice du Facteur IX et, d'autre part, du système fibrinolytique par son action activatrice du plasminogène.

La déficience en Facteur XI est héréditaire et se transmet comme un caractère autosome récessif. C'est une déficience rare mais répandue chez certaines populations d'origine moyen-orientale

Comme pour d'autres facteurs dont la déficience est rare (Facteur V, Facteur XIII, Facteur X) des produits thérapeutiques purifiés à partir de plasma humain sont encore rares ou inexistants et la seule thérapie de remplacement est effectuée avec du plasma total ou la fraction surnageante du plasma cryoprécipité mais ceci entraîne l'injection simultanée de quantités inutiles des autres protéines du plasma et donc un risque de réactions secondaires diverses importantes après des injections multiples.

Le brevet EP 512 883 B1 décrit un procédé de préparation d'un concentré de facteur XI comprenant une étape de filtration-adsorption et une étape de chromatographie sur résine échangeuse de cations. L'étape de filtration-adsorption du procédé décrit dans ce brevet est réalisée sur une série de cartouches de filtres de filtration en profondeur composés de cellulose et de perlites et portant des charges négatives ainsi que d'une faible quantité de résine chargée positivement. Ces filtres ont une porosité allant de 0,5 à 2µm, et possèdent la propriété de fixer le FXI. Le rendement de purification du FXI à cette étape est d'environ 50% et l'activité spécifique du FXI élué est d'environ 10-20 U/mg. Toutefois, en cas de rendement diminué, l'étape de chromatographie subséquente peut être fortement influencée et ainsi occasionner la non-conformité du produit final pour un défaut de concentration en FXI.

Il existe par conséquent un besoin réel de développer un procédé de purification du FXI permettant d'augmenter les rendements du procédé de purification, notamment à l'issue de l'étape d'adsorption-filtration, de sorte qu'il soit possible d'éviter tout problème de non-conformité du produit final.

### RESUME DE L'INVENTION

La présente invention concerne donc un procédé de préparation d'un concentré de facteur XI, comprenant une étape de filtration-adsorption réalisée sur un filtre composé de cellulose et de perlites ainsi que d'une faible quantité de résine chargée positivement, ledit filtre ayant un grade allant de 0,1 à 0,4 µm, de préférence de 0,2 à 0,4 µm, de préférence de 0,25 à 0,35 µm, de préférence de 0,3 µm; et une étape de chromatographie sur résine échangeuse de cations.

Lors de l'étape d'adsorption-filtration du procédé de préparation de la présente invention, l'élution du FXI adsorbé sur le filtre est effectuée au moyen d'une solution ajustée à un pH 5,5 à 6,5, comprenant par exemple du citrate de sodium, du phosphate disodique, du phosphate de potassium, de l'EDTA-disodique et du chlorure de sodium, la concentration dudit chlorure de sodium étant supérieure à 0,5 M, de préférence supérieure ou égale à 0,6 M, de préférence supérieure ou égale à 0,75 M, de préférence égale à 1M.

L'étape d'adsorption-filtration peut également comprendre un lavage du filtre, préalablement à l'élution du FXI adsorbé, avec un tampon ajusté à un pH 5,5 à 6,5 comprenant par exemple du citrate de sodium, du phosphate disodique, du phosphate de potassium, et du chlorure de sodium, la concentration dudit chlorure de sodium étant inférieure ou égale à 0,5 M, de préférence inférieure ou égale à 0,4M, de préférence égale à 0,3M.

De manière préférée, le débit de filtration de l'étape d'adsorption-filtration est compris dans la gamme allant de 7 à 20 mL/h/cm², de préférence de 10 à 15 mL/h/cm², de préférence de 12 à 14 mL/h/cm².

De manière préférée, la charge volumique utilisée à l'étape d'adsorption-filtration est comprise dans la gamme allant de 11 à 50 mL/cm², de préférence de 20 à 40 mL/cm², de préférence de 25 à 35 mL/cm².

La fraction Facteur XI résultant de l'étape d'adsorption-filtration subit une étape de diafiltration avant l'étape de purification par chromatographie. Le procédé de l'invention peut également comprendre des étapes additionnelles de concentration et d'inactivation virale entre l'étape d'adsorption-filtration et l'étape de purification par chromatographie.

Lors de l'étape de chromatographie, la résine échangeuse de cations est de préférence équilibrée avec un tampon ajusté à un pH de 5,5 à 6,5 comprenant du citrate de sodium, du chlorure de sodium, de la lysine et de l'arginine. La résine échangeuse de cations est ensuite chargée avec le FXI résultant de l'étape d'adsorption-filtration, puis lavée avec un tampon ajusté à un pH de 6,1 à 6,9 comprenant du citrate de sodium, du phosphate disodique, du phosphate de potassium, du chlorure de sodium, de la lysine et de l'arginine. Le Facteur XI est enfin élué de la résine échangeuse de cations avec un tampon ajusté à un pH de 7 à 8 comprenant du citrate de sodium, du phosphate disodique, du phosphate de potassium, du chlorure de sodium, de la lysine et de l'arginine, dans lequel la concentration de chlorure de sodium est comprise entre 0,15 et 0,20 M.

La solution de FXI résultante est préférablement stabilisée par l'addition d'antithrombine III, d'héparine et de C1-inhibiteur et peut subir à ce stade une étape d'élimination virale de préférence par filtration sur filtre de grade 15 nm. La solution de FXI est ensuite conditionnée et lyophilisée. La solution de FXI stabilisée est en particulier conditionnée en produit pharmaceutique.

La présente invention décrit également une composition de FXI susceptible d'être obtenue par le procédé de l'invention.

La demanderesse a découvert de manière surprenante que l'utilisation d'un filtre composé de cellulose et de perlites, comportant une résine chargée, et présentant un grade plus faible que les filtres utilisés dans l'état de la technique et en particulier dans le procédé du brevet EP 512 883, permet non seulement de fixer le FXI de manière plus importante, et donc d'obtenir un rendement en FXI mais aussi une pureté du FXI significativement augmentés, mais qu'il permet également d'augmenter significativement la charge volumique, le débit de filtration mis en oeuvre à cette étape, par rapport au procédé décrit dans le brevet EP 512 883. Ce filtre offre donc des avantages industriels supérieurs aux filtres utilisés dans le procédé du brevet EP 512 883 tels que diminution du nombre de cartouches filtrantes et donc une réduction de l'encombrement dans les ateliers de production, passage à grande échelle et installation et montage simplifiés et diminution de la durée globale du processus de fabrication. En effet, et de manière tout à fait inattendue, le procédé de la présente invention, qui met en oeuvre des filtres présentant un grade allant de 0,1 à 0,4 µm, de préférence de 0,2 à 0,4 µm, de préférence de 0,25 à 0,35 µm, et de préférence de 0,3 µm, facilite la fixation du FXI à l'étape d'adsorption-filtration, même lorsque la charge protéique et le débit utilisé sont significativement augmentés.

Il apparaît donc que le nouveau procédé de l'invention permet à la fois d'améliorer le rendement et l'activité spécifique du FXI purifié à l'étape d'adsorption-filtration tout en réduisant la durée de cette étape. Il en résulte une réduction des coûts et de la durée de production du FXI.

### DESCRIPTION DETAILLEE DE MODES DE REALISATION

La présente invention concerne un procédé de préparation d'un concentré de Facteur XI comprenant une étape de filtration-adsorption, retenant sélectivement le Facteur XI et une étape de chromatographie sur résine échangeuse de cations.

La première étape du procédé de purification correspond à une étape de filtration-adsorption réalisée sur filtre de filtration en profondeur. Le filtre utilisé est composé de cellulose et de perlites, et comporte une résine chargée. Le filtre utilisé possède un grade allant de 0,1 à 0,4 µm, de préférence de 0,2 à 0,4 µm, de préférence de 0,25 à 0,35 µm, et de préférence de 0,3 µm. Dans un mode de réalisation préféré, le filtre utilisé est un filtre Sartoclear® S5P (commercialisé par la société Sartorius stedim biotech GmbH), et possédant un grade de 0,3 µm. D'autres filtres comparables disponibles dans le commerce peuvent également être utilisés.

Dans un mode de réalisation particulier, le procédé de l'invention est mis en oeuvre à partir d'un surnageant de cryoprécipitation du plasma. Le surnageant utilisé dans le cadre de la présente invention peut être obtenu à partir de toute source appropriée, et correspond de préférence à du plasma d'origine humaine.

Dans un mode de réalisation préféré, le procédé de purification selon la présente invention s'applique à du surnageant de plasma humain cryoprécipité et peut être adapté à des volumes d'au moins 1.000 litres à 1.200 litres. Il permet ainsi avantageusement la production de Facteur XI pour un usage thérapeutique, à partir d'environ 1.100 litres de plasma, dans un temps assez court (environ 28 heures), y-inclus le temps éventuel de traitement d'inactivation virale (environ 8 heures).

Dans un mode de réalisation préféré, le procédé de l'invention comprend une étape d'adsorption du FXI sur le filtre de filtration en profondeur, par passage du surnageant de cryoprécipitation de plasma sur le filtre. Le filtre est ensuite avantageusement lavé avec un tampon ajusté à pH 5 à 7 et de préférence à pH 6, comprenant du citrate de sodium, du phosphate disodique, du phosphate de potassium, et du chlorure de sodium. La concentration en chlorure de sodium dans ledit tampon est de préférence inférieure ou égale à 0,3 M et de préférence égale à 0,06 M. Cette étape de filtration élimine la majorité des protéines plasmatiques.

Dans un mode de réalisation préféré, le filtre est ensuite lavé avec un tampon ajusté à pH 5 à 7 et de préférence à pH 6, comprenant du citrate de sodium, du phosphate disodique, du phosphate de potassium, et du chlorure de sodium. La concentration en chlorure de sodium dans ledit tampon est de préférence inférieure ou égale à 0,5 M, de préférence inférieure ou égale à 0,4M, de préférence égale à 0,3M. Cette étape de lavage élimine les protéines plasmatiques faiblement adsorbées sur le filtre et augmente la pureté du FXI élué à l'étape suivante.

Dans un mode de réalisation préféré, le Facteur XI qui est resté adsorbé sur le filtre est ensuite désorbé ou élué par une augmentation de la force ionique du tampon de lavage. Le Facteur XI est ainsi désorbé en utilisant un tampon dont la concentration finale en chlorure de sodium est supérieure à 0,5 M, de préférence supérieure ou égale à 0,6 M, de préférence supérieure ou égale à 0,75 M, de préférence égale à 1 M. Dans un mode de réalisation préféré, le tampon d'élution peut également comprendre une faible quantité d'inhibiteurs de protéases, avantageusement d'antithrombine III (AT III) pour protéger le Facteur XI contre l'action de protéases éventuelles et ainsi de son activation. De l'héparine et/ou du C1-inhibiteur peuvent avantageusement être ajoutés pour stabiliser le mélange obtenu.

Dans un mode de réalisation préféré de la présente invention, l'étape d'adsorption-filtration du FXI est réalisée à un débit de filtration allant de 7 à 20 mL/h/cm², de préférence de 10 à 15 mL/h/cm², de préférence de 12 à 14 mL/h/cm². Dans le contexte de la présente invention, par « débit de filtration », on entend le débit débit linéaire calculé en mesurant le volume ou le poids filtré par unité de temps et par unité de surface. Ce débit est maintenu pour toutes les étapes de l'étape adsorption/Filtration.

Dans un mode de réalisation préféré de la présente invention, l'étape d'adsorption-filtration du FXI est effectuée à une charge volumique comprise dans la gamme allant de 11 à 50 mL/cm², de préférence de 20 à 40 mL/cm², de préférence de 25 à 35 mL/cm². Dans le contexte de la présente invention, par « charge volumique », on entend le volume ou poids de solution (par exemple de cryosurnageant de précipitation) passé par unité de surface filtrante.

Dans un mode de réalisation préféré, la solution de FXI obtenue après l'étape d'adsorption-filtration est soumise à un traitement d'inactivation virale. Ce traitement d'inactivation virale est préférentiellement effectué par la méthode de traitement par solvant et/ou détergent connue de l'homme du métier. Dans un mode de réalisation préféré de l'invention, le traitement d'inactivation virale est effectué en présence de Tween 80, de préférence à une concentration finale de 1% (p/v), et de Tri-n-Butyl Phosphate ou TnBP, de préférence à une concentration finale de 0,3% (v/v). Le traitement d'inactivation virale est préférablement effectué pendant une durée d'au moins 8 heures.

Dans un mode de réalisation préféré, la solution de FXI obtenue après l'étape d'adsorption-filtration est dialysée et peut être concentrée et congelée à -80°C pour être stockée avant de procéder à l'éventuelle étape d'inactivation virale.

La solution de FXI est ensuite purifiée par chromatographie sur résine échangeuse de cations. Cette étape de chromatographie permet en plus d'éliminer totalement les produits résiduels de l'étape d'inactivation virale, lorsqu'une telle étape est mise en oeuvre. Cette étape de chromatographie permet notamment d'éliminer les protéines contaminantes subsistantes.

La fraction contenant le FXI, désorbée du filtre, dialysée et éventuellement concentrée, éventuellement congelée, et viralement inactivée, est injectée sur une colonne de chromatographie comprenant une résine échangeuse de cations. Dans un mode de réalisation préféré, la résine échangeuse de cations est plus particulièrement le Sulfate-Sepharose Fast Flow (S-Sepharose FF). Le gel S-Sepharose présente, de manière inattendue, une très haute capacité de rétention du Facteur XI (de 300 à 450 U/ml de gel), permettant par exemple d'éviter une étape ultérieure de concentration du FXI par une étape d'ultrafiltration qui entraînerait une perte de rendement. De plus, la fixation au S-Sepharose FF permet d'éluer le FXI avec un tampon ayant des propriétés comparables à un tampon physiologique, alors que d'autres résines, par exemple à base de groupements sulfopropyle, nécessitent des conditions d'élution plus drastiques qui impliquerait la mise en place d'une étape de diafiltration pour retrouver les condtions d'un produit injectable.

Dans un mode de réalisation préféré, la résine échangeuse de cations est équilibrée avec un tampon ajusté à un pH de 5,5 à 6,5, de préférence à pH 6, composé de citrate de sodium, de chlorure de sodium, de lysine et d'arginine.

Dans un mode de réalisation préféré, la résine échangeuse de cations est chargée avec la solution contenant le FXI provenant de l'étape d'adsorption-filtration qui est dialysée, et éventuellement concentrée et viralement inactivée. Un lavage est ensuite préférentiellement réalisé avec le tampon d'équilibrage de pH 5,5 à 6,5, de préférence à pH 6, composé de citrate de sodium, de chlorure de sodium, de lysine et d'arginine. Un second lavage est ensuite préférentiellement réalisé avec un tampon ajusté à un pH compris entre 6,1 et 6,9, de préférence à pH 6,5, comprenant du citrate de sodium, du phosphate disodique, du phosphate de potassium, du chlorure de sodium, de la lysine et de l'arginine.

Le Facteur XI est ensuite élué de la résine échangeuse de cations en utilisant un tampon comparable au tampon de lavage mentionné ci-dessus, mais dont le pH est compris entre 7 et 8, de préférence à pH 7,5, et dont la concentration en chlorure de sodium est de 0,15 à 0,20 M, préférentiellement de 0,17 M.
Dans un mode de réalisation préféré, le Facteur XI est stabilisé, après son élution de la résine échangeuse de cations, par l'addition de faibles quantités d'inhibiteurs de protéases, par exemple d'Antithrombine III, ainsi que d'héparine et de C1-inhibiteur. La solution de Facteur XI est stabilisée par l'addition de 0,5 à 3 UI, de préférence de 1 à 3 UI, de préférence de 2 à 3 UI d'antithrombine III, de 0,5 à 4 UI, de préférence de 1 à 4 UI, de préférence de 2 à 4 UI d'héparine et de 0,5 à 2 UI, de préférence de 1 à 2 UI, de préférence de 1,5 à 2 UI de C1-inhibiteur par 100 UI de Facteur XI.

La solution de FXI stabilisée résultante peut éventuellement subir une étape d'élimination virale de préférence par filtration sur filtre de grade 15 nm et/ou est ensuite stérilisée, par exemple par filtration, puis conditionnée et lyophilisée.

Dans un mode de réalisation préféré, la solution de Facteur XI stabilisée, et éventuellement viralement filtrée, est conditionnée en produit pharmaceutique. Ce conditionnement peut en particulier être effectué par l'ajout d'excipients pharmaceutiquement acceptables.

La présente invention décrit également une composition de Facteur XI susceptible d'être obtenue par le procédé de l'invention.

De façon avantageuse, le facteur de purification du présent procédé est au moins de 10000 par rapport au plasma de départ.

Le Facteur XI obtenu par le procédé selon la présente invention présente une activité spécifique allant de 80 à 120U/mg, de préférence au moins égale à 100 U/mg de protéines.

La haute pureté du Facteur X1 obtenu est démontrée par électrophorèse sur gel de polyacrylamide en conditions dénaturantes (en présence de SDS) et par des analyses biochimiques et son innocuité est mise en évidence par des tests biologiques sur animaux.

Le concentré de Facteur XI obtenu par le procédé selon la présente invention est donc particulièrement bien adapté à un usage thérapeutique, en particulier comme thérapie de remplacement dans les cas de déficience congénitale ou acquise en Facteur XI.

La présente invention est illustrée par les exemples et les modes de réalisation décrits et représentés, mais ne saurait y être limitée dans la mesure où elle est susceptible de nombreuses variantes accessibles à l'homme de l'art.

### EXEMPLE 1 : COMPOSITION DE FACTEUR XI

### - Matériau de départ

Chaque lot de Facteur XI est préparé à partir d'un volume d'environ 1000 litres de surnageant de cryoprécipité de plasma humain. Le surnageant de cryoprécipitation est d'abord clarifié sur un filtre ayant un grade de 1 µm, tel que le filtre Profile® II (Pall).

### - Première étape de purification

Le surnageant du cryoprécipité est passé sur un filtre Sartoclear® S5P (Sartorius), ayant un grade de 0,3 µm, à un débit de 13 mL/h/cm² (soit environ 14,3 L/h). La charge volumique utilisée correspond à 30 mL de surnageant de cryoprécipitation /cm² de surface filtrante, et l'ensemble de la filtration est réalisé à une température de 2 à 8°C.

Après élimination du filtrat qui contient une majorité des protéines du surnageant du cryoprécipité, le filtre est lavé avec du tampon comprenant du citrate de sodium 5mM, du phosphate disodique 5mM, du phosphate de potassium 5 mM, du chlorure de sodium 0,065 M, et ajusté à un pH de 6 avec de l'acide citrique.

Le FXI est, à ce stade de la filtration, adsorbé sur le filtre.

Une étape de lavage plus stringent est réalisée avec un tampon comparable au tampon de lavage mais contenant 0,3M de NaCl.

Le Facteur XI est élué du filtre en augmentant la force ionique du tampon de lavage. Le tampon d'élution utilisé, additionné d'EDTA-disodique, contient ainsi 1M NaCl. 0,2 U/ml d'antithrombine III sont ajoutés à la solution de FXI résultante pour protéger le facteur XI de l'action des protéases plasmatiques résiduelles. L'EDTA-disodique du tampon participe également à cette action protectrice.

La solution de FXI ainsi récupérée est concentrée environ 30 fois et dialysée pour éliminer l'EDTA, à l'aide d'un système d'ultrafiltration Pall présentant un seuil de coupure à 10 kD. Le débit exprimé est un débit linéaire. Il peut être calculé en mesurant le volume ou le poids filtré par unité de temps et par unité de surface. Ce débit est maintenu pour toutes les étapes de l'étape adsorption/Filtration. L'étape de dialyse permet également de placer la fraction de FXI dans les conditions d'osmolalité et de composition chimique du tampon d'équilibrage de la colonne de S-Sépharose FF afin de garantir la fixation du FXI sur la colonne.

Le tampon de dialyse est composé de citrate de sodium 5 mM, de chlorure de sodium 140 mM, de L-lysine 5,5 mM et d'arginine 20mM, et ajusté à pH 6.

La solution dialysée est passée sur un filtre 0,45 µm KA2NLP (Pall) + filtre 0.22 µm KA2NFP1 (Pall) pour clarifier la solution et éventuellement éliminer les contaminants bactériens présents.

### - Traitement d'inactivation virale

La solution contenant le Facteur XI est soumise à un traitement par solvant-détergent connu pour son efficacité contre les virus à enveloppe lipidique (Horowitz et al. 1985, Transfusion 25, 516-522) et qui comprend une incubation de 8 heures à 25°C en présence de 0,3 % de tri-n-butyl-phosphate (TnBP) et de 1 % de Tween 80.

### - Deuxième étape de purification

Le but de cette étape est d'augmenter la pureté du Facteur XI (en éliminant essentiellement le Facteur II, le Facteur VII, le Facteur X, les Kininogènes de haut poids moléculaire, l'Activateur de la Prékallicréine, les Immunoglobulines M, les Immunoglobulines G, la Fibronectine et le Fibrinogène, tout en éliminant la solution de Solvant -Détergent utilisée pour le traitement d'inactivation virale.

On utilise une colonne de chromatographie contenant une résine échangeuse de cations, plus particulièrement le gel "Sulfate-Sepharose Fast Flow" (S Sepharose FF, fabriqué et distribué par Pharmacia, Uppsala-Suède).

La colonne est équilibrée avec le tampon de dialyse décrit plus haut.
Après avoir chargé la solution protéique sur la colonne, celle-ci est rincée avec au moins 20 volumes de tampon de dialyse pH 6,0 décrit plus haut pour éliminer les protéines non adsorbées sur le gel et les agents d'inactivation virale Le gel est ensuite lavé avec une solution tampon comprenant du citrate de sodium 10 mM, du phosphate disodique 5 mM, du phosphate de potassium 5 mM, du chlorure de sodium 92 mM, de la lysine 27 mM et de l'arginine 11,5 mM, pH 6,5 pour éluer les protéines faiblement adsorbées sur le gel.

Le Facteur XI est élué de la colonne par une augmentation du pH du tampon à 7,5 et une augmentation de la concentration en NaCl à 170 mM.
Le débit linéaire de passage des solutions tampon d'équilibrage, de lavages et d'élution est de 50 cm/h.

La solution FXI éluée est additionnée de trois stabilisants:
- de l'antithrombine III, dans une quantité d'environ 3 UI d'Antithrombine pour 100 UI de FXI,
- du C1-Inhibiteur, dans une quantité d'environ 2 U de C1-Inhibiteur pour 100 UI de FXI, et
- de l'héparine, dans une quantité d'environ 4 UI Héparine pour 100 UI de FXI.

La solution stabilisée est filtrée 0,22 µm sur Millipak 20, distribuée (10 ml par flacon) et lyophilisée. Celle-ci peut être filtrée sur filtre de grade 15 nm avant filtration 0,22 µm et répartition en flacons.

### - Analyses biochimiques et biologiques du concentré de Facteur XI

Les rendements (%), puretés du FXI (UI/mg) et « niveaux d'activation » mesurés par le rapport Facteur XI activé/Facteur XI (FXIa/FXI), obtenus aux différentes étapes unitaires du procédé sont illustrés dans le tableau 1 suivant.

**Tableau 1**

| Etapes | Moyenne de 3 lots |
|---|---|
| Cryosurnageant | |
| | |
| FXI (UI/mL) | 0,74 |
| FXI (Total UI) | 23660 (100%) |
| Activité Spécifique (UI/mg) | 0,01 |
| FXIa/FXI (µg/UI) | 0,236 |
| Eluat Sartoclear | |
| | |
| FXI (UI/mL) | 4,1 |
| FXI (Total UI) | 17763 (82%) |
| Activité Spécifique (UI/mg) | 31,3 |
| FXIa/FXI (µg/UI) | 3,1 |
| Après Ultrafiltration | |
| | |
| FXI (UI/mL) | 85,1 |
| FXI (Total UI) | 15630 (74%) |
| Activité Spécifique (UI/mg) | 23,3 |
| FXIa/FXI (µg/UI) | ND |
| Eluat S-Sepharose | |
| | |
| FXI (UI/mL) | 121 |
| FXI (Total UI) | 12126 (55%) |
| Activité Spécifique (UI/mg) | 292 |
| FXIa/FXI (µg/U) | 0,46 |
| Stabilisé | |
| | |
| FXI (UI/mL) | 102,7 |
| Activité Spécifique (UI/mg) | 95,7 |
| FXIa/FXI (µg/U) | <0,001 |

Le procédé de l'invention permet d'obtenir un rendement de filtration/adsorption du FXI d'environ 80%, et une pureté de FXI d'environ 20 à 40 UI/mg. A titre de comparaison, le procédé décrit dans le brevet EP 512 883 permet d'obtenir un rendement de filtration/adsorption d'environ 50% et une pureté de FXI d'environ 10 à 20 UI/mg.

Le rendement et pureté obtenus dans le cadre de la présente invention se révèle donc significativement supérieur à celui obtenu lors de la mise en oeuvre du procédé de l'état de la technique. En outre, le procédé de l'invention a été mis en oeuvre dans des conditions de fixation et d'élution du FXI dans lesquelles la charge protéique et le débit de filtration sont respectivement augmentés d'un facteur 3 et d'un facteur 2 par rapport aux conditions mise en oeuvre avec le procédé du brevet EP 512 883.

Il en résulte que le procédé de l'invention permet de réduire significativement les coûts et la durée de production du FXI, tout en fournissant un facteur XI ayant un niveau de pureté supérieur à celui décrit dans l'état de la technique pour l'étape de filtration/adsorption.

L'élimination des protéines présentes dans le surnageant de cryoprécipitation au cours du procédé de purification est illustrée dans le tableau 2 suivant. Les résultats ont été comparés aux données obtenues avec le procédé du brevet EP 512 883.

### Elimination des Protéines au cours de la purification du FXI

| **Eluat filtre en profondeur selon l'invention** | | |
|---|---|---|
| | Solution de F XI obtenue par le procédé de l'invention (moyenne de 3 lots) | Solution de FXI obtenue par le procédé de EP 0 512 883 (moyenne de 3 lots) |
| Protéines (g/L) | 3,8 | 8,2 |
| Kininogènes de haut poids moléculaire (µg/mL) | ≥ 300 | 418 |
| Facteur II (UI/mL) | 1,04 | 8,8 |
| Immunoglobulines G (mg/mL) | 0,28 | 0,9 |
| Immunoglobulines M (mg/mL) | 0,21 | 0,26 |
| Fibronectine (mg/mL) | 0,018 | 0,09 |
| Composant C3 du complément (mg/mL) | 0,041 | 0,1 |
| C1-Inhibiteur (mg/mL) | 0,011 | 0,029 |
| α-2 Macroglobuline (mg/mL) | 0,039 | 0,17 |

| **Eluat S-Sepharose FF** | | |
|---|---|---|
| | Solution de FXI obtenue par le procédé de l'invention (moyenne de 3 lots) | Solution de FXI obtenue par le procédé de EP 0 512 883 (moyenne de 3 lots) |
| Protéines (g/L) | 0,42 | 0,64 |
| Kininogènes de haut poids moléculaire (µg/mL) | < 10 | 123 |
| Facteur II (UI/mL) | < 0,1 | < 0,126 |
| Immunoglobulines G (mg/mL) | 0,003 | 0,02 |
| Immunoglobulines M (mg/mL) | 0,011 | 0,022 |
| Fibronectine (mg/mL) | <0,003 | 0,03 |
| Composant C3 du complément (mg/mL) | <0,008 | < 0,03 |
| C1-Inhibiteur (mg/mL) | 0,01 | 0,044 |
| α-2 Macroglobuline (mg/mL) | <0,003 | < 0,01 |

| **Eluat S-Sepharose FF stabilisé** | | |
|---|---|---|
| Protéines (g/L) | 1,1 | 0,73 |
| Kininogènes de haut poids moléculaire (µg/mL) | < 10 | 49 |
| Facteur II (UI/mL) | < 0,1 | < 0,126 |
| Immunoglobulines G (mg/mL) | 0,004 | 0,018 |
| Immunoglobulines M (mg/mL) | 0,01 | 0,01 |
| Fibronectine (mg/mL) | <0,004 | 0,01 |
| Composant C3 du complément (mg/mL) | <0,008 | < 0,03 |
| C1-Inhibiteur (mg/mL) | 0,52* | 0,53* |
| α-2 Macroglobuline (mg/mL) | <0,003 | < 0,01 |

| | | |
|---|---|---|
| * après ajout pour stabilisation | | |

L'absence de contamination résiduelle par des facteurs de la coagulation et des constituants du système kinine (kininogènes) est soigneusement contrôlée selon les méthodes classiques.

Après reconstitution du produit lyophilisé des tests classiques de tolérance sur animaux sont exécutés :
- test de thrombogénicité sur lapins
- test d'hypotension sur rats
- test de toxicité sur souris.

Les tests sur lapins démontrent que la composition selon l'invention n'est pas thrombogène puisque la DE 50 (dose efficace 50) est supérieure à 1000 U FXI/kg alors que cette même valeur est de 40 à 60 U/kg pour un concentré de PPSB (fraction coagulante composée de proconvertine, prothrombine, facteur Stuart, facteur antihémophilique B) qui est donc beaucoup plus thrombogène, et peut entraîner effectivement des phénomènes de thromboses et de coagulation intravasculaire disséminée à forte dose chez l'homme.

La composition de FXI selon l'invention n'induit pas de phénomènes d'hypotension quand il est injecté par voie intraveineuse chez le rat à la dose de 50 U FXI/kg. Ce modèle animal est très sensible à la présence de composants plasmatiques à propriétés vasoactives et démontre l'absence de ces composants dans la composition de facteur XI obtenu par le procédé décrit.

Injecté par voie intraveineuse chez la souris à une forte dose (2500 U/kg) il n'induit aucune létalité ni aucune perturbation du comportement sur une période de 7 jours.

## Revendications

1. Procédé de préparation d'un concentré de facteur XI, comprenant :
a) une étape de filtration-adsorption réalisée sur un filtre comprenant de la cellulose et des perlites ainsi qu'une résine chargée, ledit filtre possédant un grade allant de 0,1 à 0,4 µm, de préférence de 0,2 à 0,4 µm, de préférence de 0,25 à 0,35 µm, de préférence de 0,3 µm; et
b) une étape de chromatographie sur résine échangeuse de cations.

2. Procédé selon la revendication 1, dans lequel l'étape a) comprend l'élution du FXI adsorbé sur le filtre au moyen d'une solution ajustée à un pH 5,5 à 6,5, comprenant du citrate de sodium, du phosphate disodique, du phosphate de potassium, de l'EDTA-disodique et chlorure de sodium, la concentration dudit chlorure de sodium étant supérieure à 0,5 M, de préférence supérieure ou égale à 0,6 M, de préférence supérieure ou égale à 0,75 M, de préférence égale à 1M.

3. Procédé selon la revendication 2, dans lequel l'étape a) comprend un lavage du filtre, préalablement à l'élution du FXI adsorbé, avec un tampon ajusté à un pH 5,5 à 6,5 comprenant du citrate de sodium, du phosphate disodique, du phosphate de potassium, et du chlorure de sodium, la concentration dudit chlorure de sodium étant inférieure ou égale à 0,5 M, de préférence inférieure ou égale à 0,4M, de préférence égale à 0,3M.

4. Procédé selon l'une quelconque des revendications 1 à 3 comprenant une étape additionnelle d'inactivation virale entre l'étape a) et l'étape b), et/ou une étape d'élimination virale après l'étape b).

5. Procédé selon l'une quelconque des revendications 1 à 4, dans lequel la résine échangeuse de cations de l'étape b) est équilibrée avec un tampon ajusté à un pH de 5,5 à 6,5 comprenant du citrate de sodium, du chlorure de sodium, de la lysine et de l'arginine.

6. Procédé selon l'une quelconque des revendications 1 à 5, dans lequel la résine échangeuse de cations de l'étape b) est chargée avec le FXI résultant de l'étape a), puis lavée avec un tampon ajusté à un pH de 6,1 à 6,9 comprenant du citrate de sodium, du phosphate disodique, du phosphate de potassium, du chlorure de sodium, de la lysine et de l'arginine.

7. Procédé selon l'une quelconque des revendications 1 à 6, **caractérisé en ce que** la solution de Facteur XI obtenue à l'étape b) est stabilisée par l'addition de 0,5 à 3 UI d'antithrombine III, de 0,5 à 4 UI d'héparine et de 0,5 à 2 UI de C1-inhibiteur par 100 UI de Facteur XI.

8. Procédé selon l'une quelconque des revendications 1 à 7, comprenant en outre une étape de conditionnement de la solution stabilisée de FXI en produit pharmaceutique.

9. Procédé selon l'une quelconque des revendications 1 à 8, comprenant en outre une étape finale de lyophilisation de la solution de Facteur XI.

## Patentansprüche

1. Verfahren zur Herstellung eines Faktor XI-Konzentrats, umfassend:
a) einen Filtrations-Adsorptions-Schritt, der an einem Filter, welches Cellulose und Perlite sowie ein beladenes Harz umfasst, durchgeführt wird, wobei das Filter einen Grad von 0,1 bis 0,4 µm, vorzugsweise von 0,2 bis 0,4 µm, vorzugsweise von 0,25 bis 0,35 µm, vorzugsweise von 0,3 µm aufweist, und
b) einen Schritt einer Kationenaustauscherharz-Chromatographie.

2. Verfahren nach Anspruch 1, bei dem Schritt a) die Elution des an dem Filter adsorbierten FXI mittels einer auf einen pH-Wert von 5,5 bis 6,5 eingestellten Lösung, die Natriumcitrat, Dinatriumphosphat, Kaliumphosphat, Dinatrium-EDTA und Natriumchlorid enthält, umfasst, wobei die Konzentration an Natriumchlorid größer als 0,5 M, vorzugsweise größer als oder gleich 0,6 M, vorzugsweise größer als oder gleich 0,75 M, vorzugsweise gleich 1 M ist.

3. Verfahren nach Anspruch 2, bei dem Schritt a) vor der Elution des adsorbierten FXI das Waschen des Filters mit einem auf einen pH-Wert von 5,5 bis 6,5 eingestellten Puffer, der Natriumcitrat, Dinatriumphosphat, Kaliumphosphat und Natriumchlorid enthält, umfasst, wobei die Konzentration an Natriumchlorid geringer als oder gleich 0,5 M, vorzugsweise geringer als oder gleich 0,4 M, vorzugsweise gleich 0,3 M ist.

4. Verfahren nach einem der Ansprüche 1 bis 3, umfassend einen zusätzlichen Schritt zur Virusinaktivierung zwischen Schritt a) und Schritt b) und/oder einen Schritt zur Viruselimination nach Schritt b).

5. Verfahren nach einem der Ansprüche 1 bis 4, bei dem das Kationenaustauscherharz des Schrittes b) mit einem auf einen pH-Wert von 5,5 bis 6,5 eingestellten Puffer, der Natriumcitrat, Natriumchlorid, Lysin und Arginin umfasst, ins Gleichgewicht gebracht wird.

6. Verfahren nach einem der Ansprüche 1 bis 5, bei dem das Kationenaustauscherharz des Schrittes b) mit dem aus Schritt a) hervorgehenden FXI beladen wird, dann mit einem auf einen pH-Wert von 6,1 bis 6,9 eingestellten Puffer, der Natriumcitrat, Dinatriumphosphat, Kaliumphosphat, Natriumchlorid, Lysin und Arginin umfasst, gewaschen wird.

7. Verfahren nach einem der Ansprüche 1 bis 6, **dadurch gekennzeichnet, dass** die in Schritt b) erhaltene Faktor XI-Lösung durch die Zugabe von 0,5 bis 3 IU Antithrombin III, von 0,5 bis 4 IU Heparin und von 0,5 bis 2 IU von C1-Inhibitor pro 100 IU Faktor XI stabilisiert wird.

8. Verfahren nach einem der Ansprüche 1 bis 7, ferner umfassend einen Schritt des Konditionierens der stabilisierten FXI-Lösung als pharmazeutisches Produkt.

9. Verfahren nach einem der Ansprüche 1 bis 8, ferner umfassend einen abschließenden Schritt der Gefriertrocknung der Faktor XI-Lösung.

## Claims

1. A method for preparing a concentrate of Factor XI, comprising
a) a filtration-adsorption step performed on a filter comprising cellulose and perlites and a charged resin, the said filter having a grade ranging from 0.1 to 0.4 µm, preferably 0.2 to 0.4 µm, preferably 0.25 to 0.35 µm, and preferably of 0.3 µm; and
b) a chromatography step on cation exchange resin.

2. The method according to claim 1 wherein step a) comprises eluting of the FXI adsorbed on the filter, using a solution adjusted to a pH of 5.5 to 6.5 comprising sodium citrate, disodium phosphate, potassium phosphate, disodium EDTA and sodium chloride, the concentration of said sodium chloride being higher than 0.5 M, preferably 0.6 M or higher, preferably 0.75 M or higher, and preferably it is 1 M.

3. The method according to claim 2 wherein step a) comprises washing of the filter prior to eluting the adsorbed FXI, using a buffer adjusted to a pH of 5.5 to 6.5 comprising sodium citrate, disodium phosphate, potassium phosphate and sodium chloride, the concentration of said sodium chloride being equal to lower than 0.5 M, preferably 0.4 M or lower, preferably it is 0.3 M.

4. The method according to any of claims 1 to 3 comprising an additional step for viral inactivation between step a) and step b), and/or a virus removal step after step b).

5. The method according to any of claims 1 to 4 wherein the cation exchange resin of step b) is equilibrated with a buffer adjusted to a pH of 5.5 to 6.5 comprising sodium citrate, sodium chloride, lysine and arginine.

6. The method according to any of claims 1 to 5 wherein the cation exchange resin of step b) is loaded with the FXI resulting from step a), then washed with a buffer adjusted to a pH of 6.1 to 6.9 comprising sodium citrate, disodium phosphate, potassium phosphate, sodium chloride, lysine and arginine.

7. The method according to any of claims 1 to 6 **characterized in that** the solution of Factor XI obtained at step b) is stabilised through the addition of 0.5 to 3 IU Antithrombin III, 0.5 to 4 IU of heparin and 0.5 to 2 IU of C1-inhibitor per 100 IU of Factor XI.

8. The method according to any of claims 1 to 7 further comprising a step to package the stabilised FXI solution as pharmaceutical product.

9. The method according to any of claims 1 to 8 further comprising a final lyophilisation step of the Factor XI solution.
